# EUROPÄISCHE PATENTSCHRIFT

(11) **EP 3 426 668 B1**
(45) Veröffentlichungstag und Bekanntmachung des Hinweises auf die Patenterteilung: **21.04.2021**
(21) Anmeldenummer: 17706520.8
(22) Anmeldetag: 27.02.2017
(51) Int. Cl.: C07F 15/00, C07C 211/63, B01J 23/42, C01G 55/00, B01J 35/04, B01J 37/03

(54) **EDELMETALLVERBINDUNGEN**
NOBLE METAL COMPOUNDS
RACCORDS EN METAL PRECIEUX

(30) Priorität: 26.02.2016 EP 16157675
(43) Veröffentlichungstag der Anmeldung: 16.01.2019
(60) Teilanmeldung: 21163126.2
(73) Patentinhaber: Umicore AG & Co. KG, 63457 Hanau-Wolfgang (DE)
(72) Erfinder: WOERNER, Eileen, 61130 Nidderau (DE); EBERT, Timo, 63796 Kahl (DE); LENNARTZ, Michael, 60435 Frankfurt (DE); KARCH, Ralf, 63801 Kleinostheim (DE); DOPPIU, Angelino, 63500 Seligenstadt (DE); RIVAS NASS, Andreas, 64625 Bensheim (DE); FREY, Annika, 63457 Hanau (DE)
(86) Internationale Anmeldenummer: PCT/EP2017/054529
(87) Internationale Veröffentlichungsnummer: WO 2017/144726

(56) Entgegenhaltungen:
- EP-A2- 1 175 948
- WO-A1-2008/151731
- WO-A1-2015/168327
- WO-A2-2008/145386
- CN-A- 103 864 855
- CN-A- 103 880 889
- ZHENGZHENG YANG ET AL: "Size-dependent CO and propylene oxidation activities of platinum nanoparticles on the monolithic Pt/TiO 2 -YO x diesel oxidation catalyst under simulative diesel exhaust conditions", CATALYSIS SCIENCE & TECHNOLOGY, Bd. 5, Nr. 4, 2015, Seiten 2358-2365, XP055295839, United Kingdom ISSN: 2044-4753, DOI: 10.1039/C4CY01384K

## Beschreibung

Bei der Abscheidung von Platinmetall auf Trägermaterialien, wie z.B. Oxidpartikeln, werden an die platinhaltigen Platin-Ausgangsverbindungen mehrere Anforderungen gestellt.

EP 1175948 zeigt die Verbindung [EtNH₃]₂[Pt(OH)₆] und die Verwendung dieser Verbindung zur Herstellung trägergestützter Katalysatoren, wie Elektroden-Katalysatoren in Brennstoffzellen

CN 103864855 zeigt die Verbindung Pt-EAH der Formel [HO-CH₂-CH₂-NH3][Pt(OH)6] sowie Verfahren zur Herstellung aus H2Pt(OH)6 und EA in Wasser sowie die Verwendung von Pt-EAH als Precursor zur Herstellung von trägergestützten Katalysatoren.CN 103880889 zeigt die Verbindung Pt-EAH sowie Verfahren zur Herstellung aus H₂Pt(OH)₆ und EA sowie die Verwendung von Pt-EAH als Vorläuferverbindung zur Herstellung von trägergestützten Katalysatoren mit geringem Chlorid und Nitrat-Gehalt.

ZhengZheng Yang et al., Catalysis Science & Technology , Bd. 5, Nr. 4, 2015, Seiten 2358-2365 zeigt die Verwendung von Pt-EAH als Vorläuferverbindung zur Herstellung trägergestützter Platin-Katalysatoren zur Dieselmotor-Abgasbehandlung.

WO 2008/151731 zeigt Tetramethylammonium Hexahydroxoplatinat der Formel [(NMe₄)⁺Pt(OH)₆] sowie die Verwendung als Vorläuferverbindung zur Herstellung heterogener Katalysatoren durch Beschichtung eines Trägerkörpers, ein Verfahren zur Herstellung von Katalysatoren durch Imprägnieren des Formkörpers sowohl mit einer sauren wässrigen Metallsalzlösung als auch mit einer basischen wässrigen Lösung, wobei die Basenlösung die Metallkomplexe ausfällt.

WO 2008/145386 zeigt Tetraalkylammonium Hexahydroxoplatinat der Formel [NMe₄Pt(OH)₆] sowie einen heterogenen Katalysator enthaltend [NMe₄Pt(OH)₆] und Verfahren zur Herstellung von Katalysatoren durch Imprägnieren des Formkörpers sowohl mit [NMe₄Pt(OH)₆] als auch mit einer Carbonsäure- oder Carbonsäuresalz-Lösung, wobei die Metallkomponente ausfällt.

WO 2015/168327 zeigt die Ammonium-Hydroxoplatinate [(NMe₄)₂Pt(OH)₆] und [(HO-CH2-CH2-NH3)2Pt(OH)6] zur Beschichtung von Katalysatoren.

Diese platinhaltigen Platin-Ausgangsverbindungen sollen vorteilhaft in basischen Lösungen mit einem Platingehalt von mindestens etwa 10% (wie z.B. 9 bis 11%) hergestellt werden können, halogenfrei oder zumindest halogenarm sein, mit Ausnahme von Kohlenstoff, Wasserstoff, Sauerstoff, und Stickstoff keine Fremdelemente enthalten, einen möglichst niedrigen Stickstoffgehalt aufweisen, um Stickoxidemissionen zu minimieren. Zudem sollen sie durch Veränderung des pH Werts möglichst vollständig ausgefällt werden können.

Diese Vorgaben werden von Tetraalkylammonium- Tetra- oder Hexahydroxometallaten der Formel (N(Alkyl)₄)_{y}[M(OH)ₓ], wobei
M Platin ist;
Alkyl eine Alkylgruppe ausgewählt aus der Gruppe bestehend aus Ethyl, Propyl, Isopropyl, n-Butyl, Isobutyl, tert.-Butyl, n-Pentyl, sec-Pentyl, 3-Pentyl, 2-Methylbutyl, Isopentyl, 3-Methylbut-2-yl, 2-Methylbut-2-yl, Neopentyl, n-Hexyl, 3-Methylpentyl, Isohexyl, Neohexyl, 2,3,-Dimethylbutyl oder deren Kombinationen ist;
Y 2 ist; und
X 4 oder 6 ist.

Die Alkylgruppen können gleich oder verschieden sein, vorteilhaft sind sie gleich.

Bewährt hat sich insbesondere Tetraethylammonium-hexahydroxoplatinat, (N(CH₂CH₃)₄)₂[Pt(OH)₆], nachfolgend als Pt-TEAH bezeichnet. Die Verbindungen können als Lösung mit der gewünschten Platinkonzentration hergestellt werden. Der Gehalt an Halogen (insbesondere Chlor) wird über den Chlorgehalt der verwendeten Edukte eingestellt.

Dabei handelt es sich insbesondere um die folgenden Einzelverbindungen der allgemeinen Formel (N(Alkyl)₄)_{y}[M(OH)ₓ], wobei Alkyl, M und x die folgenden Bedeutungen gemäß Formel 1 aufweisen:

| Nummer | M | X | Y | Alkyl |
|---|---|---|---|---|
| 1 | Platin | 6 | 2 | Ethyl |
| 2 | Platin | 6 | 2 | Propyl |
| 3 | Platin | 6 | 2 | Isopropyl |
| 4 | Platin | 6 | 2 | n-Butyl |
| 5 | Platin | 6 | 2 | Isobutyl |
| 6 | Platin | 6 | 2 | tert.-Butyl |
| 7 | Platin | 6 | 2 | n-Pentyl |
| 8 | Platin | 6 | 2 | sec-Pentyl |
| 9 | Platin | 6 | 2 | 3-Pentyl |
| 10 | Platin | 6 | 2 | 2-Methylbutyl |
| 11 | Platin | 6 | 2 | Isopentyl |
| 12 | Platin | 6 | 2 | 3-Methylbut-2-yl |
| 13 | Platin | 6 | 2 | 2-Methylbut-2-yl |
| 14 | Platin | 6 | 2 | Neopentyl |
| 15 | Platin | 6 | 2 | n-Hexyl |
| 16 | Platin | 6 | 2 | 3-Methylpentyl |
| 17 | Platin | 6 | 2 | Isohexyl |
| 18 | Platin | 6 | 2 | Neohexyl |
| 19 | Platin | 6 | 2 | 2,3,-Dimethylbutyl |

Während Hexahydroxoplatinate eine gut definierte Struktur aufweisen ist die Struktur bei anderen Metallhydroxiden nicht immer eindeutig charakterisiert bzw. charakterisierbar.

Im Allgemeinen können die Verbindungen auch in Strukturen wie (N(Alkyl)₄)ₛ [M(OH)V(H₂O)_{w}]^{u-v}, wobei u die Ladung des Metalls, v = 1 bis Koordinationszahl des Metalls, w = Koordinationszahl des Metalls -v und s = 1 bis u-v sein kann.

Die beschriebenen Verbindungen, Tetraalkylammonium- Tetra- bzw. Hexa-hydroxometallate der Formel (N(Alkyl)₄)_{y}[M(OH)ₓ], können durch Umsetzung von Hydroxoverbindungen des Metalls wie etwa Tetra- oder Hexahydroxosäuren der jeweiligen Metalle mit den jeweiligen Tetraalkylammoniumhydroxiden, gegebenenfalls in einem Reaktionsmedium erhalten werden.

Als Hydroxoverbindung insbesondere geeignet ist im Fall von Platin Hexahydroxoplatinsäure. Diese Verbindung kann durch Zugabe von Metallsalzen zu einer Base gefällt und so erhalten werden. Als Basen geeignet sind Alkalimetallhydroxide bzw. deren wässrige Lösungen, aber auch die ohnehin verwendeten Tetraalkylammoniumhydroxide, wie speziell Tetraethylammoniumhydroxid. Ist ein möglichst niedriger Halogengehalt gewünscht, so ist natürlich das Nitrat dem Halogenid vorzuziehen.

Diese Verbindungen sind nicht immer eindeutig charakterisierbar und können teilweise auch Aquo-Liganden aufweisen. Alle solchen Verbindungen sind hier kollektiv mit Hydroxoverbindungen eines Metalls wie etwa deren Tetra- oder Hexahydroxosäuren gemeint, als welche die Ausgangsverbindungen auch aufgefasst werden können.

Pt-TEAH, Tetraethylammonium-hexahydroxoplatinat, kann zum Beispiel durch Umsetzung von Hexahydroxoplatinsäure mit Tetraethylammoniumhydroxid, optional in Gegenwart eines Reaktionsmediums, hergestellt werden.

Hexahydroxoplatinsäure kann mit verschiedenen Halogengehalten, insbesondere Chlorgehalten, hergestellt werden. Diese liegen im Allgemeinen bei 30.000 ppm oder weniger, oder 20.000 ppm oder weniger, oder 15.000 ppm oder weniger, insbesondere 10.000 ppm oder weniger, 5.000 ppm oder weniger oder 1.000 ppm oder weniger, wobei sich der Halogengehalt auf Platinmetall bezieht.

In einer Ausgestaltung ist die Zugabe der Edukte Hexahydroxoplatinsäure und Tetraethylammoniumhydroxid unkritisch, weshalb Tetraethylammoniumhydroxid oder dessen Lösung vorgelegt und danach Hexahydroxoplatinsäure zugegeben werden kann oder aber es können zunächst Hexahydroxoplatinsäure vorgelegt und anschließend Tetraethylammoniumhydroxid oder deren Lösung zugegeben und anschließend miteinander umgesetzt werden.

Optional kann die Umsetzung in einem Reaktionsmedium erfolgen, welches vorteilhaft ein Lösemittel sein kann. Gut geeignet sind polare, protische Lösemittel.

Das Reaktionsmedium kann ausgewählt sein aus der Gruppe bestehend aus Wasser, ein oder mehrere Alkohole, eine oder mehrere Säuren und deren Mischungen. Insbesondere kann das Reaktionsmedium ein Lösemittel ausgewählt aus der Gruppe bestehend aus Wasser, Methanol, Ethanol, Propanol, Isopropanol, Butanol, 1-Methylbutanol, 2-Methylbutanol, tert.-Butanol, Ameisensäure, Essigsäure, Propionsäure und deren Kombinationen sein. Besonders geeignet als Reaktionsmedium ist Wasser; in der Praxis wird in diesem Fall üblicherweise demineralisiertes Wasser bzw. destilliertes Wasser verwendet.

Vorteilhaft wird das Reaktionsmedium vor Zugabe der Edukte vorgelegt. Es kann jedoch auch gleichzeitig mit einem Edukt vorgelegt werden, beispielsweise wenn ein Edukt oder beider Edukte als Lösung oder Suspension vorgelegt bzw. zugegeben wird, wie zum Beispiel eine Lösung von Tetraethylammoniumhydroxid.

Die Edukte werden für eine vorbestimmte Reaktionszeit und bei einer in einem vorbestimmten Bereich liegenden Reaktionstemperatur umgesetzt.

Im Allgemeinen ist eine Reaktionszeit von 10 Minuten bis 24 Stunden ausreichend, insbesondere eine Reaktionszeit von 20 Minuten bis 12 Stunden, 30 Minuten bis 6 Stunden, 45 Minuten bis 3 Stunden oder 45 Minuten bis 120 Minuten, oder 50 Minuten bis 70 Minuten. Meist ist eine Reaktionszeit von etwa einer Stunde ausreichend.

Die Reaktionstemperatur kann von 10°C bis 100°C, insbesondere 15°C bis 80°C oder 20°C bis 50°C oder 20°C bis 40°C betragen. Wird ein Reaktionsmedium eingesetzt, so liegt die Reaktionstemperatur maximal bei der Siedetemperatur des Reaktionsmediums. In der Praxis bewährt haben sich Temperaturen von 20°C bis 50°C.

Zur Herstellung der gewünschten Tetraalkylammonium- Tetra- oder Hexa-hydroxometallate der Formel (N(Alkyl)₄)_{y}[M(OH)ₓ] werden mindestens 0,5, insbesondere mindestens 1,7 Moläquivalente Tetraalkylammoniumhydroxid pro Mol der Hydroxoverbindungen des Metalls wie etwa Tetra- oder Hexahydroxosäuren des Metalls eingesetzt. Bewährt haben sich 0,5 Moläquivalente bis 17 Moläquivalente, 1,7 Moläquivalente bis 17 Moläquivalente, insbesondere 2 Moläquivalente bis 6 Moläquivalente Tetraalkylammoniumhydroxid pro Mol der Hydroxoverbindungen des Metalls wie etwa Tetra-oder Hexahydroxosäuren des Metalls. Ist das verwendete Metall M Palladium, so sind insbesondere 0,5 bis 2 Moläquivalente, insbesondere 1 bis 1,7 Moläquivalente Tetraalkylammoniumhydroxid vorteilhaft

Zur Herstellung von beispielsweise Pt-TEAH werden mindestens 1,7 Moläquivalente Tetraethylammoniumhydroxid pro Mol Hexahydroxoplatinsäure eingesetzt. Bewährt haben sich 1,7 Moläquivalente bis 17 Moläquivalente, insbesondere 2 Moläquivalente bis 6 Moläquivalente Tetraethylammoniumhydroxid pro Mol Hexahydroxoplatinsäure.

Das gewünschte Produkt (Tetraalkylammonium- Tetra- oder Hexa-hydroxometallat der Formel (N(Alkyl)₄)_{y}[M(OH)ₓ] fällt bei Verwendung eines Reaktionsmediums als Lösung an, insbesondere als wässrige Lösung, wobei die Konzentration an Platin von 1 Gew.-% bis 15 Gew.-%, insbesondere 10 Gew.-% bis 15 Gew.-% beträgt.

Ist das gewünschte Produkt Pt-TEAH, so fällt es bei Verwendung eines Reaktionsmediums als Lösung an, insbesondere als wässrige Lösung, wobei die Platinkonzentration von 1 Gew.-% bis 15 Gew.-%, insbesondere 10 Gew.-% bis 15 Gew.-% beträgt.

Die Tetraalkylammonium- Tetra- oder Hexa-hydroxometallate der Formel (N(Alkyl)₄)_{y}[M(OH)ₓ] bzw. deren Zubereitungen können als Precursor zur Herstellung heterogener Katalysatoren (wie z.B. Autoabgaskatalysatoren), für den Einsatz in galvanischen Bädern, als Vorprodukt für die Herstellung homogener Katalysatoren oder auch selbst als homogener Katalysator verwendet werden. Ebenfalls geeignet sind diese zur Herstellung von Materialien für Brennstoffzellen.

Insbesondere Pt-TEAH bzw. dessen Zubereitungen sind als Precursor zur Herstellung heterogener Katalysatoren (wie z.B. Autoabgaskatalysatoren oder Brennstoffzellen), für den Einsatz in galvanischen Bädern, als Vorprodukt für die Herstellung homogener Katalysatoren oder auch selbst als homogener Katalysator gut geeignet.

Zur Herstellung eines heterogenen Katalysators kann eine Lösung eines Tetraalkylammonium- Tetra- oder Hexa-hydroxometallate der Formel (N(Alkyl)₄)_{y}[M(OH)ₓ] mit einem Trägermaterial wie einem Metalloxid wie zum Beispiel Aluminiumoxid oder Siliziumdioxid vermischt werden. Das Metalloxid, also Aluminiumoxid oder Siliziumdioxid, wird vorteilhaft in Form von Pulver oder Partikel eingesetzt.

Anschließend wird das Tetraalkylammonium- Tetra- oder Hexa-hydroxometallat der Formel (N(Alkyl)₄)_{y}[M(OH)ₓ] umgesetzt, d.h. einer chemischen Reaktion unterworfen, wobei eine Metallverbindung entsteht, gegebenenfalls ausfällt und sich zumindest teilweise auf der Oberfläche der Trägermaterialpartikel, also der Metalloxidpartikel, abscheidet. Die Abscheidung kann somit durch Auffällen, aber auch durch Adsorption erfolgen.

Unter bestimmten, geeigneten Bedingungen kann auch das elementare Metall abgeschieden werden. Hierzu muss ein Reduktionsmittel zugefügt werden, wie beispielsweise Wasserstoff, Hydrazin oder Ameisensäure. Dieses Verfahren ist insbesondere zur Herstellung von Materialien für Brennstoffzellen von Interesse.

Die Umsetzung und das Ausfällen der Metallverbindung kann durch Änderung des pH-Wertes erfolgen. Hierbei kann insbesondere eine Hydroxoverbindung des jeweiligen Metalls wie etwa die Tetrahydroxosäure oder Hexahydroxosäure des Metalls (also von Rhodium, Platin, Iridium oder Palladium) ausgefällt und auf den Metalloxidpartikeln abgeschieden werden.

Durch Kalzinieren der so erhaltenen Metalloxidpartikel, auf denen die Metallverbindung abgeschieden wurde, kann in einem weiteren Verfahrensschritt elementares Metall auf den Partikeln des Trägermaterials erzeugt werden.

In einer spezifischen Ausführungsform kann zur Herstellung eines heterogenen Katalysators eine Lösung von Pt-TEAH mit einem Trägermaterial wie einem Metalloxid, wie z.B. Aluminiumoxid oder Siliziumdioxid, vermischt werden, welches vorteilhaft in Form von Pulver oder Partikeln eingesetzt wird. Anschließend wird das Pt-TEAH umgesetzt, d.h. einer chemischen Reaktion unterworfen, wobei eine Platinverbindung ausfällt und sich zumindest teilweise auf der Oberfläche der Trägermaterialpartikel, also der Metalloxidpartikel, abscheidet.

Die Umsetzung und das Ausfällen der Platinverbindung kann durch Änderung des pH-Wertes erfolgen. Hierbei kann insbesondere Hexahydroxoplatinsäure ausgefällt und auf den Metalloxidpartikeln abgeschieden werden.

Durch Kalzinieren der so erhaltenen Metalloxidpartikel, auf denen die Platinverbindung abgeschieden wurde, kann in einem weiteren Verfahrensschritt elementares Platin auf den Partikeln des Trägermaterials erzeugt werden.

Sowohl Trägermaterial, auf dem die Metallverbindung, insbesondere eine Platinverbindung abgeschieden wurde als auch Trägermaterial, auf dem die Metallverbindung, insbesondere eine Platinverbindung durch Kalzinierung zu elementarem Metall, insbesondere metallischem Platin umgesetzt wurde, können zur Herstellung von Autoabgaskatalysatoren verwendet werden.

Hierzu wird eine Beschichtungssuspension (auch als Washcoat bezeichnet) hergestellt, welche das Trägermaterial enthält. Hierbei handelt es sich wie oben beschrieben meist um Metalloxide, insbesondere also Aluminiumoxid oder Siliziumdioxid.

Mit dieser Beschichtungssuspension werden die Innenwände der Strömungskanäle der Tragkörper, welche zur Herstellung der Autoabgaskatalysatoren erforderlich sind, auf an sich bekannte Weise beschichtet, wie z.B. in EP-A1-1273344 beschrieben.

Die Tragkörper werden meist aus Metall oder Keramik hergestellt und können als Durchflußwabenkörper oder Wandflußfilter ausgestaltet sein. Hierbei handelt es sich im Allgemeinen um zylindrische Körper, die eine umlaufende Mantelfläche und zwei gegenüberliegende Stirnflächen aufweisen, wobei die Tragkörper von Strömungskanälen durchzogen sind, die zwischen beiden Stirnflächen verlaufen.

Weit verbreitet sind Katalysatorkörper mit rundem Querschnitt, elliptischem oder dreieckförmigem Querschnitt. Die Strömungskanäle weisen meist einen quadratischen Querschnitt auf und sind in einem engen Raster über den gesamten Querschnitt der Katalysatorkörper angeordnet. Je nach Anwendungsfall variiert die Kanal- beziehungsweise Zelldichte der Strömungskanäle meist zwischen 10 und 250 cm⁻². Für die Abgasreinigung von Personenkraftwagen werden heute noch oft Katalysatortragkörper mit Zelldichten von etwa 62 cm⁻² eingesetzt. Bei Wandflussfiltern sind diese alternierend verschlossen, so dass ein durchfließender Abgasstrom durch die Poren der Wandungen gelenkt wird, welche die Strömungskanäle bilden.

Nach dem Aufbringen der Beschichtungssuspension wird der beschichtete Wabenkörper gegebenenfalls weiteren Verfahrensschritten unterworfen, getrocknet und schließlich kalziniert. Wird Trägermaterial, auf dem die Platinverbindung abgeschieden wurde, verwendet, so findet die Umwandlung in elementares Platin dann bei diesem Kalzinierungsschritt nach Beschichtung des Tragkörpers statt.

Wurde bereits ein nach Abscheidung der Platinverbindung kalziniertes Trägermaterial (wie z.B. Platin auf einem Aluminiumoxid-Trägermaterial) zur Herstellung der Beschichtungssuspension verwendet, so liegt bereits vor der Beschichtung des Tragkörpers elementares Platin auf dem Trägermaterial vor und die Kalzinierung des beschichteten Tragkörpers ist hinsichtlich der Umwandlung der abgeschiedenen Platinverbindung ohne Effekt.

### Beispiele

Beispiel 1: Herstellung von Pt-TEAH (Tetraethylammonium-hexahydroxoplatinat)

In einem Reaktor mit einem Füllvolumen von fünf Litern wurden 758 g demineralisiertes Wasser vorgelegt und 1,47 kg wässrige Tetraethylammoniumhydroxidlösung mit einer Konzentration von 35 Gew.-% zugegeben, entsprechend etwas 2 mol pro mol Platin.

Unter Rühren wurden dann 894,74g Hexahydroxoplatinsäure mit einem Gehalt von 38 g Platin zugegeben. Es wurde anschließend bei einer Temperatur von ca. 20°C bis 30°C eine Stunde nachgerührt, wobei eine gelborange Lösung entstand, die über eine G4-Glasfritte filtriert und analysiert wurde.

Die Lösung enthielt einen Edelmetallgehalt von 10,9 Gew.-% Platin und einen pH-Wert von 13,9.

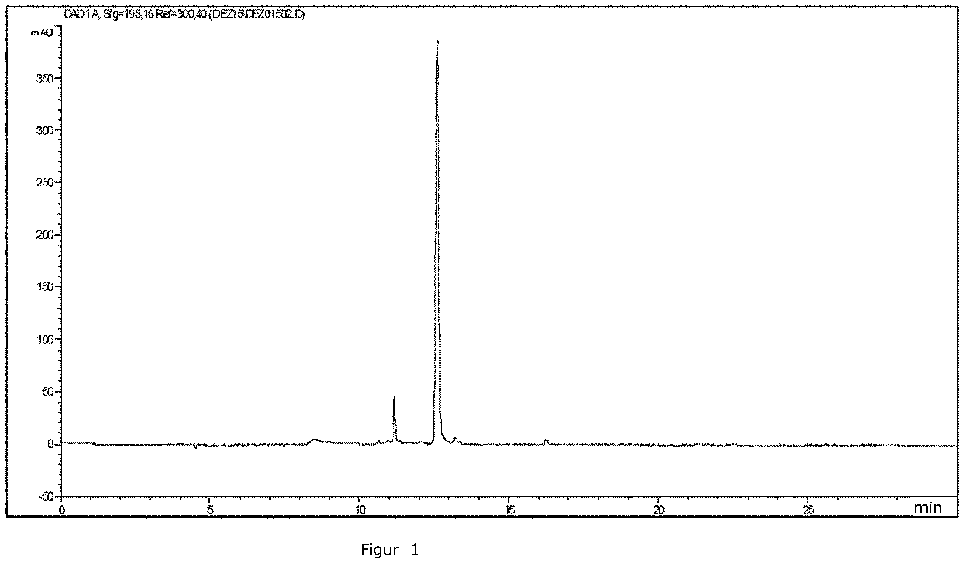

Das Produkt wurde auch durch Kapillarelektrophorese untersucht, das Ergebnis ist in Figur 1 gezeigt. Die Zusammensetzung ist charakteristisch für die Verbindung Pt-TEAH. Der Hauptpeak entspricht dem [Pt(OH)₆]²⁻ Komplex. Der kleinere, zweite Peak entspricht einem weiteren anionischen Pt-Komplex der unbekannten Zusammensetzung [Pt(OH)₆₋ₓ(NEt)ₓ]^{2-x} (mit x=1-2). Die prozentuale Verteilung beider Hauptkomponenten kann je nach Herstellmethode und Eduktqualität schwanken.

Beispiel 2: Herstellung von Pt-TPAH (Tetrapropylammonium-hexahydroxoplatinat; Alkyl = n-Propyl)

Es wurde verfahren wie in Beispiel 1, allerdings wurde eine wässrige Lösung von Tetrapropylammoniumhydroxid (40 Gew.-%) eingesetzt und eine wässrige Lösung enthaltend 31,92 Gew.-% Hexahydroxoplatinsäure vorgelegt und für 180 Minuten bei 20°C bis 25°C gerührt, danach wurde auf 50°C erwärmt, weitere 5 ml der Tetrapropylammoniumhydroxidlösung zugegeben, über Nacht (ca. 18 Stunden) stehen gelassen und über eine G4-Glasfritte filtriert und 15 Minuten zentrifugiert. Die erhaltene klare Tetrapropylammonium-hexahydroxoplatinat Lösung wurde wies einen Platingehalt von 9,08% auf.

Beispiel 3: Herstellung von Pt-TBAH (Tetrabutylammonium-hexahydroxoplatinat; Alkyl = n-Butyl)

Es wurde verfahren wie in Beispiel 2, allerdings wurde gefrorenes Tetrabutylammoniumhydroxid^{∗}30 H₂O eingesetzt, eine wässrige Lösung enthaltend 39,3 Gew.-% Hexahydroxoplatinsäure vorgelegt und über Nacht (ca. 18 Stunden) bei 20°C bis 25°C gerührt und 15 Minuten zentrifugiert. Die erhaltene klare Tetrabutylammonium-hexahydroxoplatinat-Lösung wies eine dunkelgelbe Farbe auf.

Die Lösung enthielt einen Edelmetallgehalt von 9,8 Gew.-% und einen pH-Wert von etwa 10 (Bestimmung mit Universalindikatorpapier).

Beispiel 4: Herstellung einer Beschichtungssuspension zur Herstellung eines heterogenen Katalysators
45 g Aluminiumoxid und 16 g eines Zeolithen (Zeolite-HSZ-0940NHA von Tosoh) wurden in Wasser suspendiert und für 15 Minuten gerührt. Anschließend wurde durch Zugabe von Tetraethylammoniumhydroxid der pH-Wert auf 10 eingestellt, dann wurden 0,81 g gelöstes Platin in Form einer löslichen Platinverbindung zugegeben und für 15 Minuten nachgerührt. Anschließend wurden 0,54 g Palladium in Form von Palladiumnitrat in Wasser zugegeben und erneut für 15 Minuten nachgerührt. Anschließend wurde mit Essigsäure der pH-Wert auf einen Wert von 5 eingestellt und 5 Minuten nachgerührt. Anschließend wurde der Gehalt an Edelmetall bestimmt, der noch in Wasser gelöst vorlag.

Vergleichsbeispiel 4a: Das Platin wurde in Form einer Ethanolamin-Hexahydroxoplatinatlösung in Wasser zugegeben. Es wurde festgestellt, dass nach Abschluss des Versuchs 10% der ursprünglichen Palladiumkonzentration und 20% der ursprünglichen Palladiumkonzentration gelöst vorlagen. Das restliche Edelmetall war auf den vorhandenen Träger (Aluminiumoxid und Zeolith) aufgefällt.

Beispiel 4b: Das Platin wurde in Form einer wässrigen Lösung von Tetraethylammonium-hexahydroxoplatinat (Pt-TEAH) zugegeben. Nach Abschluss des Versuchs wurde festgestellt, dass sich kein Platin mehr in Lösung befand und lediglich 0,8% der ursprünglichen Palladiumkonzentration noch in Lösung befanden. Das restliche Edelmetall war auf den vorhandenen Träger (Aluminiumoxid und Zeolith) aufgefällt.

## Patentansprüche

1. Ein Tetraalkylammonium-Tetra- oder Hexa-hydroxometallat der Formel (N(Alkyl)₄)_{y}[M(OH)ₓ], wobei
M ein Platin ist;
Alkyl eine Alkylgruppe ausgewählt aus der Gruppe bestehend aus Ethyl, Propyl, Isopropyl, n-Butyl, Isobutyl, tert.-Butyl, n-Pentyl, sec-Pentyl, 3-Pentyl, 2-Methylbutyl, Isopentyl, 3-Methylbut-2-yl, 2-Methyl-but-2-yl, Neopentyl, n-Hexyl, 3-Methylpentyl, Isohexyl, Neohexyl, 2,3,-Dimethylbutyl oder deren Kombinationen ist;
Y 2 ist; und
X 4 oder 6 ist.

2. Tetraethylammonium-hexahydroxoplatinat nach Anspruch 1, wobei Alkyl = Ethyl und X = 6 ist.

3. Verfahren zur Herstellung von Tetraalkylammonium- Tetra- oder Hexahydroxometallaten nach Anspruch 1 oder 2 durch Umsetzung von Hydroxoverbindungen von Platin mit Tetraalkylammoniumhydroxid, optional in Gegenwart eines Reaktionsmediums.

4. Verfahren nach Anspruch 3, wobei ein Tetraalkylammoniumhydroxid oder deren Lösung vorgelegt, Hydroxoverbindungen eines Metalls wie etwa Tetra-oder Hexahydroxosäuren zugegeben und für eine vorbestimmte Reaktionszeit und bei einer in einem vorbestimmten Bereich liegenden Reaktionstemperatur umgesetzt wird.

5. Verfahren nach Anspruch 3, wobei Hydroxoverbindungen eines Metalls wie etwa Tetra-oder Hexahydroxosäuren vorgelegt, Tetraalkylammoniumhydroxid oder deren Lösung zugegeben und für eine vorbestimmte Reaktionszeit und bei einer in einem vorbestimmten Bereich liegenden Reaktionstemperatur umgesetzt wird.

6. Verfahren nach einem oder mehreren der Ansprüche 3 bis 5, wobei das Reaktionsmedium vor Zugabe der Reaktanden vorgelegt wird.

7. Verfahren nach einem oder mehreren der Ansprüche 3 bis 6, wobei das Reaktionsmedium ein polares, protisches Lösemittel ist.

8. Verfahren nach einem oder mehreren der Ansprüche 3 bis 7, wobei das Reaktionsmedium ausgewählt ist aus der Gruppe bestehend aus Wasser, ein oder mehrere Alkohole, eine oder mehrere Säuren und deren Mischungen.

9. Verfahren nach einem oder mehreren der Ansprüche 3 bis 8, wobei das Reaktionsmedium ein Lösemittel ausgewählt aus der Gruppe bestehend aus Wasser, Methanol, Ethanol, Propanol, Isopropanol, Butanol, 1-Methylbutanol, 2-Methylbutanol, tert.- Butanol, Ameisensäure, Essigsäure, Propionsäure und deren Kombinationen ist.

10. Verfahren nach einem oder mehreren der Ansprüche 3 bis 9, wobei das Verfahren bei einer Temperatur von 10°C bis 100°C, insbesondere 15°C bis 80°C oder 20°C bis 50°C oder 20°C bis 40°C durchgeführt wird.

11. Verfahren nach einem oder mehreren der Ansprüche 3 bis 10, wobei 0,5 bis 17 oder 1,7 bis 17, insbesondere 2 bis 6 Moläquivalente Tetraethylammoniumhydroxid pro Mol Hydroxoverbindungen des Metalls wie etwa Tetra-oder Hexahydroxosäuren eingesetzt werden.

12. Wäßrige Lösung von Tetraalkylammonium-Tetra- oder Hexa-hydroxometallat nach Anspruch 1 oder 2 mit einer Platinkonzentration von 1 Gew.-% bis 15 Gew.-%.

13. Verwendung von Tetraalkylammonium-Tetra- oder Hexa-hydroxometallat nach einem oder mehreren der Ansprüche 1 bis 2 oder deren wäßrige Zubereitung als Precursor zur Herstellung heterogener Katalysatoren, Autoabgaskatalysatoren, Materialien für Brennstoffzellen, für den Einsatz in galvanischen Bädern, als Vorprodukt für die Herstellung homogener Katalysatoren oder als homogener Katalysator.

14. Verfahren zur Herstellung eines Autoabgaskatalysators, wobei eine Suspension enthaltend Tetraalkylammonium-Tetra- oder Hexa-hydroxometallat nach einem oder mehreren der Ansprüche 1 bis 2, insbesondere Tetraethylammonium-hexahydroxoplatinat (Pt-TEAH), (N(CH2CH3)4)2[Pt(OH)6], und mindestens ein Trägermaterial erzeugt wird, durch Umsetzen des Tetraalkylammonium-Tetra- oder Hexahydroxometallats das Metall als Hydroxoverbindungen wie etwa Tetra-oder Hexahydroxosäure auf das Trägermaterial gefällt und ein Katalysator-Tragkörper ganz oder teilweise mit dem Trägermaterial beschichtet wird und die auf das Trägermaterial aufgebrachte Hexahydroxoplatinsäure vor oder nach der Beschichtung des Katalysator-Tragkörpers kalziniert wird.

15. Verfahren nach Anspruch 14, wobei das Trägermaterial ein Metalloxid, insbesondere Aluminiumoxid oder Siliziumdioxid ist.

16. Verfahren nach Anspruch 14 oder 15, wobei der Katalysator-Tragkörper ein Wandflußfilter oder ein Durchflußwabenkörper ist.

17. Verfahren nach einem oder mehreren der Ansprüche 14 bis 16, wobei das Umsetzen des Tetraalkylammonium-Tetra- oder Hexahydroxometallats durch Änderung des pH-Wertes erfolgt.

18. Verfahren nach einem oder mehreren der Ansprüche 14 bis 17, wobei das Metall Platin ist, welches als Hexahydroxoplatinsäure gefällt wird

19. Verfahren nach einem oder mehreren der Ansprüche 17 bis 21, wobei das Umsetzen von Tetraethylammonium-hexahydroxoplatinat (Pt-TEAH) zum Fällen von Hexahydroxoplatinsäure auf das Trägermaterial durch Änderung des pH-Wertes erfolgt.

## Claims

1. A tetraalkylammonium tetra- or hexa-hydroxometallate of the formula (N(Alkyl)₄)_{y}[M(OH)ₓ], wherein
M is a platinum;
alkyl is an alkyl group selected from the group consisting of ethyl, propyl, isopropyl, n-butyl, isobutyl, tert.-butyl, n-pentyl, sec-pentyl, 3-pentyl, 2-methylbutyl, isopentyl, 3-methylbut-2-yl, 2-methyl-but-2-yl, neopentyl, n-hexyl, 3-methylpentyl, isohexyl, neohexyl, 2,3,-dimethylbutyl, or combinations thereof;
Y is 2; and
X is 4 or 6.

2. Tetraethylammonium hexahydroxoplatinate according to claim 1, wherein alkyl = ethyl and X = 6.

3. Method for producing tetraalkylammonium tetra- or hexa-hydroxometallates according to claim 1 or 2 by reacting hydroxo compounds of platinum with tetraalkylammonium hydroxide, optionally in the presence of a reaction medium.

4. Method according to claim 3, wherein a tetraalkylammonium hydroxide or the solution thereof is provided, hydroxo compounds of a metal such as tetra- or hexahydroxo acids are added, and these are reacted for a predetermined reaction time and at a reaction temperature within a predetermined range.

5. Method according to claim 3, wherein hydroxo compounds of a metal such as tetra- or hexahydroxo acids are provided, tetraalkylammonium hydroxide or the solution thereof is added, and these are reacted for a predetermined reaction time and at a reaction temperature within a predetermined range.

6. Method according to one or more of claims 3 to 5, wherein the reaction medium is provided before addition of the reactants.

7. Method according to one or more of claims 3 to 6, wherein the reaction medium is a polar, protic solvent.

8. Method according to one or more of claims 3 to 7, wherein the reaction medium is selected from the group consisting of water, one or more alcohols, one or more acids, and mixtures thereof.

9. Method according to one or more of claims 3 to 8, wherein the reaction medium is a solvent selected from the group consisting of water, methanol, ethanol, propanol, isopropanol, butanol, 1-methylbutanol, 2-methylbutanol, tert.-butanol, formic acid, acetic acid, propionic acid, and combinations thereof.

10. Method according to one or more of claims 3 to 9, wherein the method is implemented at a temperature of 10°C to 100°C, in particular 15°C to 80°C, or 20°C to 50°C, or 20°C to 40°C.

11. Method according to one or more of claims 3 to 10, wherein 0.5 to 17 or 1.7 to 17, in particular 2 to 6, molar equivalents of tetraethylammonium hydroxide are used per mole of hydroxo compounds of the metal, such as tetra- or hexahydroxo acids.

12. Aqueous solution of tetraalkylammonium tetra- or hexa-hydroxometallate according to claim 1 or 2, having a platinum concentration of 1% by weight to 15% by weight.

13. Use of tetraalkylammonium tetra- or hexa-hydroxometallate according to one or more of claims 1 to 2, or the aqueous preparation thereof, as a precursor for producing heterogeneous catalysts, automobile exhaust catalysts, materials for fuel cells, for use in galvanic baths, as a precursor for the production of homogeneous catalysts, or as a homogeneous catalyst.

14. Method for producing an automobile exhaust gas catalyst, wherein a suspension containing tetraalkylammonium tetra- or hexa-hydroxometallate according to one or more of claims 1 to 2, in particular tetraethylammonium hexahydroxoplatinate (Pt-TEAH), (N(CH₂CH₃)₄)₂[Pt(OH)₆], and at least one carrier material is generated; by reacting the tetraalkylammonium tetra- or hexa-hydroxometallate, the metal is precipitated on the carrier material as hydroxo compounds such as tetra- or hexahydroxo acids, and a catalyst carrier body is completely or partially coated with the carrier material, and the hexahydroxoplatinic acid applied to the carrier material is calcined before or after the coating of the catalyst carrier body.

15. Method according to claim 14, wherein the carrier material is a metal oxide, in particular aluminum oxide or silicon dioxide.

16. Method according to claim 14 or 15, wherein the catalyst carrier body is a wall-flow filter or a flow-through honeycomb body.

17. Method according to one or more of claims 14 to 16, wherein the reaction of the tetraalkylammonium tetra- or hexa-hydroxometallate is effected by changing the pH value.

18. Method according to one or more of claims 14 to 17 wherein the metal is platinum which is precipitated as a hexahydroxoplatinic acid.

19. Method according to one or more of claims 17 to 21, wherein the reaction of tetraethylammonium hexahydroxoplatinate (Pt-TEAH) to precipitate hexahydroxoplatinic acid on the carrier material is effected by changing the pH value.

## Revendications

1. Tétrahydroxométallate ou hexahydroxométallate de tétraalkylammonium de formule (N(alkyle)₄)_{y}[M(OH)ₓ], dans lequel
M représente platine ;
alkyle représente un groupe alkyle choisi dans le groupe constitué par l'éthyle, le propyle, l'isopropyle, le n-butyle, l'isobutyle, le tert-butyle, le n-pentyle, le sec-pentyle, le 3-pentyle, le 2-méthylbutyle, l'isopentyle, le 3-méthylbut-2-yle, le 2-méthyl-but-2-yle, le néopentyle, le n-hexyle, le 3-méthylpentyle, l'isohexyle, le néohexyle, le 2,3-diméthylbutyle ou leurs combinaisons ;
y est égal à 2 et
x est égal à 4 ou 6.

2. Hexahydroxoplatinate de tétraéthylammonium selon la revendication 1, dans lequel alkyle = éthyle et x = 6.

3. Procédé de préparation de tétrahydroxométallates ou d'hexahydroxométallates de tétraalkylammonium selon la revendication 1 ou 2 par réaction des composés hydroxo du platine avec de l'hydroxyde de tétraalkylammonium, éventuellement en présence d'un milieu réactionnel.

4. Procédé selon la revendication 3, dans lequel un hydroxyde de tétraalkylammonium ou sa solution est fourni, des composés hydroxo d'un métal, tels que par exemple des acides tétrahydroxo ou hexahydroxo sont ajoutés et, pendant un temps de réaction prédéfini et à une température de réaction se trouvant dans une plage prédéfinie, sont mis en réaction.

5. Procédé selon la revendication 3, dans lequel des composés hydroxo d'un métal tels que par exemple des acides tétrahydroxo ou hexahydroxo sont fournis, l'hydroxyde de tétraalkylammonium ou sa solution est ajouté et, pendant un temps de réaction prédéfini et à une température de réaction se trouvant dans une plage prédéfinie, est mis en réaction.

6. Procédé selon l'au moins une des revendications 3 à 5, dans lequel le milieu réactionnel est fourni avant l'ajout des réactifs.

7. Procédé selon l'au moins une des revendications 3 à 6, dans lequel le milieu réactionnel est un solvant polaire protique.

8. Procédé selon l'au moins une des revendications 3 à 7, dans lequel le milieu réactionnel est choisi dans le groupe constitué par l'eau, au moins un alcool, au moins un acide et leurs mélanges.

9. Procédé selon l'au moins une des revendications 2 à 8, dans lequel le milieu réactionnel est un solvant choisi dans le groupe constitué par l'eau, le méthanol, l'éthanol, le propanol, l'isopropanol, le butanol, le 1-méthylbutanol, le 2-méthylbutanol, le tert-butanol, l'acide formique, l'acide acétique, l'acide propionique ou leurs combinaisons.

10. Procédé selon l'au moins une des revendications 3 à 9, dans lequel ledit procédé est mis en œuvre à une température comprise entre 10 °C et 100 °C, en particulier entre 15 °C et 80 °C ou entre 20 °C et 50 °C ou entre 20 °C et 40 °C.

11. Procédé selon l'au moins une des revendications 3 à 10, dans lequel 0,5 à 17, ou 1,7 à 17, en particulier 2 à 6, équivalents molaires d'hydroxyde de tétraéthylammonium par mole de composés hydroxo du métal, tels que par exemple des acides tétrahydroxo ou hexahydroxo, sont utilisés.

12. Solution aqueuse de tétrahydroxométallate ou d'hexahydroxométallate de tétraalkylammonium selon la revendication 1 ou 2, présentant une concentration en platine comprise entre 1 % en poids et 15 % en poids.

13. Utilisation d'un tétrahydroxométallate ou d'un hexahydroxométallate de tétraalkylammonium selon l'au moins une des revendications 1 à 2 ou de sa préparation aqueuse en tant que précurseur pour la préparation de catalyseurs hétérogènes, de catalyseurs des gaz d'échappement automobiles, de matériaux pour piles à combustible, pour l'utilisation dans des bains galvaniques, en tant que précurseur pour la préparation de catalyseurs homogènes ou en tant que catalyseur homogène.

14. Procédé de préparation d'un catalyseur des gaz d'échappement automobiles, dans lequel une suspension contenant du tétrahydroxométallate ou de l'hexahydroxométallate de tétraalkylammonium selon l'au moins une des revendications 1 à 2, en particulier de l'hexahydroxoplatinate de tétraéthylammonium (Pt-TEAH), (N(CH₂CH₃)₄)₂[Pt(OH)₆], et au moins un matériau support est généré, par mise en réaction du tétrahydroxométallate ou de l'hexahydroxométallate de tétraalkylammonium, le métal est précipité sous forme de composé hydroxo, tel que par exemple un acide tétrahydroxo ou hexahydroxo, sur le matériau support et un corps support catalytique est revêtu totalement ou partiellement par le matériau support et l'acide hexahydroxoplatinique appliqué sur le matériau support est calciné avant ou après le revêtement du corps support catalytique.

15. Procédé selon la revendication 14, dans lequel le matériau support est un oxyde métallique, en particulier l'oxyde d'aluminium ou le dioxyde de silicium.

16. Procédé selon la revendication 14 ou 15, dans lequel le corps support catalytique est un filtre à parois filtrantes ou un corps en nid d'abeille à écoulement.

17. Procédé selon l'au moins une des revendications 14 à 16, dans lequel la mise en réaction du tétrahydroxométallate ou de l'hexahydroxométallate de tétraalkylammonium est effectuée par modification de la valeur de pH.

18. Procédé selon l'au moins une des revendications 14 à 17, dans lequel le métal est le platine, lequel est précipité sous forme d'acide hexahydroxoplatinique.

19. Procédé selon l'au moins une des revendications 17 à 21, dans lequel la mise en réaction de l'hexahydroxoplatinate de tétraéthylammonium (Pt-TEAH) pour la précipitation de l'acide hexahydroxoplatinique sur le matériau support est effectuée par modification de la valeur de pH.
